# EUROPÄISCHE PATENTSCHRIFT

(11) **EP 1 684 722 B1**
(45) Veröffentlichungstag und Bekanntmachung des Hinweises auf die Patenterteilung: **31.12.2008**
(21) Anmeldenummer: 04765912.3
(22) Anmeldetag: 09.10.2004
(51) Int. Cl.: A61K 9/127

(54) **MEDIKAMENTÖSE GEZIELTE LOKALE LIPOLYSE**
MEDICAMENTOUSLY TARGETED LOCAL LIPOLYSIS
LIPOLYSE LOCALE CIBLEE AU MOYEN DE MEDICAMENTS

(30) Priorität: 24.10.2003 DE 10349979
(43) Veröffentlichungstag der Anmeldung: 02.08.2006
(73) Patentinhaber: Merz Pharma GmbH & Co. KGaA, 60318 Frankfurt (DE)
(72) Erfinder: BODERKE, Peter, 85824 Schwalbach (DE); POOTH, Rainer, 63303 Dreieich-Götzenhain (DE); SANDOW, Juergen, 61479 Glashütten (DE); GOSSEL, Matthias, 65719 Hofheim (DE); NIETSCH, Karl-Heinz, 41470 Neuss (DE); SATTLER, Gerhard, 64287 Darmstadt (DE); VOGEL, Gerhard, 73430 Aalen (DE)
(74) Vertreter: Michalski Hüttermann & Partner Patentanwälte
(86) Internationale Anmeldenummer: PCT/EP2004/011320
(87) Internationale Veröffentlichungsnummer: WO 2005/041919

(56) Entgegenhaltungen:
- EP-A- 0 470 437
- EP-A- 0 615 746
- WO-A-02/098436
- US-A1- 2004 151 786

## Beschreibung

Die Erfindung betrifft wässrige Phospholipidsysteme, enthaltend mindestens ein Phospholipid, mindestens eine Gallensäure und Wasser, die sich zur Herstellung von Arzneimitteln zur Behandlung von Fettgewebserkrankungen eignen und zur Regression des krankhaft proliferierten Fettgewebes führen.

Wässrige phospholipidische Phospholipidsysteme sind für verschiedene Anwendungen bekannt. So werden diese Systeme beispielsweise im kosmetischen Bereich oder für die Herstellung von pharmazeutischen Produkten eingesetzt. Diese Systeme sind dadurch ausgezeichnet, dass sie kugelförmige Vesikel aufweisen, die auch als Liposomen bezeichnet werden. Die genannten Liposomen sind durch eine Lipiddoppelmembran nach außen hin begrenzt und enthalten in ihrem Inneren eine wässrige Phase. Wässrige Phospholipidsysteme, enthaltend mindestens ein Phospholipid, mindestens eine Gallensäure und Wasser, werden beispielsweise in der Europäischen Patentanmeldung EP 0 615 746 beschrieben. Im Handel befindet sich das Produkt Lipostabil® N i.V. (Rote Liste, März 2003), welches ein wässriges Phospholipidsystem darstellt, das Phospholipide, Gallensäure, DL-alpha-Tocopherol, Ethanol und Wasser enthält und zur Prophylaxe und Behandlung von Fettembolien zugelassen ist.

Es wird davon berichtet, dass Fettpölsterchen wie sie bei übergewichtigen Menschen unter den Augen, am Bauch oder an Hüften auftreten schrumpfen und es zu ästhetischen Verbesserungen im Aussehen der behandelten Personen gekommen sein soll, wenn diese Personen subkutane Injektion von Lipostabil® N i.V. erhielten (Patricia Guedes Rittes, The Use of Phosphatidylcholine for Correction of Lower Lid Bulging Due to Prominent Fat Pads, Dermatol Surg 2001 ;27: 391-392).

In dem Bestreben, wirksame Verbindungen zur Behandlung von Erkrankungen des Fettgewebes zu finden, wurde nun gefunden, dass das erfindungsgemäß eingesetzte Liposomensystem zur Regression von krankhaft proliferierten Fettgewebe führt. Es kommt zu einer Lipolyse des Fettgewebes und der betroffene krankhaft proliferierte Fettgewebsbereich wird zurückgebildet. Wie unten ausgeführt, handelt es sich bei diesen Erkrankungen nicht nur um ästhetisch entstellende Proliferationen des Fettgewebes, sondern um schmerzhafte Zustände und Beeinträchtigungen der Körperfunktionen.

Die Erfindung betrifft daher die Verwendung von wässrigen Phospholipidsystemen, enthaltend
a) mindestens ein Phospholipid,
b) mindestens eine Gallensäure und
c) Wasser
zur Herstellung eines Arzneimittels zur Behandlung von Fettgewebserkrankungen.

Ein weiterer Gegenstand der Erfindung ist die Verwendung des genannten wässrigen Liposomensystems zur Herstellung eines Arzneimittels zur Rückbildung von Fettgewebsgeschwülsten.

Unter dem Begriff "Phospholipid" werden Verbindungen wie 3-sn-Phosphatidylcholin, Soja (Phospholipon 90), 3-sn-Phosphatidylcholin, reduziertes Soja (Phospholipon 90H), 3-(3sn)-Phospohatidyl)glycerol Soja (Phospholipon G), Dimyristoylphosphatidylglycerol, Lyso-phosphatidylcholin oder Dipalmitoylphosphatidylglycerol, sowie deren physiologisch verträglichen Salze verstanden.

Unter dem Begriff "Gallensäure" werden Verbindungen wie Desoxycholsäure, Cholsäure, Lithocholsäure, Chenodesocycholsäure, Hyodesoxycholsäure, Trihydroxykoprostansäure, Ursodesoxycholsäure, Taurocholsäure oder Glykocholsäure, sowie deren physiologisch verträglichen Salze verstanden.

Unter dem Begriff "Fettgewebserkrankungen" werden beispielsweise folgende Erkrankungen verstanden:

**Lipome** sind Fettgewebsgeschwülste, dies sind gutartige, langsam wachsende, meist kugelige, eventuell gestielte (= L. pendulum) oder gar zottige (= L. arborescens, beispielsweise der Gelenkzotten) mesenchymale Geschwülste aus - vergrößerten - Fettgewebszellen, bevorzugt im Unterhautzellgewebe, eventuell zentral verknöchernd (= L. ossificans), verschleimend (= L. myxomatodes) oder verkalkend (= L. petrificans), auch mit vermehrter Bindegewebs- und Kapselbildung (= L. fibrosum), Blutgefäßneubildung (= L. teleangiectodes), selten maligne entartend (= L. sarcomatodes, Liposarkom). Sie sind als krankhaft einzustufen, da sie wachsen und ihre bindegewebige Hülle an sich schmerzhaft sein kann, ebenso wie die von ihnen ausgehende Kompression auf Blutgefäße, die Nerven-Schmerzen verursachen kann.

**Morbus Dercum,** genannt Lipomatosis dolorosa ist eine Sonderform der hypertrophen Proliferation von Fettgewebe, welches sich zwischen der dermalen Fettfaszie (Kampa'sche Fettfaszie) und der Unterseite der Dermis befindet. Durch hormonelle Einflüsse kommt es zur verstärkten Wasserbindungskapazität dieser Fettzellen, die selbst wiederum durch Druckphänomene Lymphbahnstauungen im Bereich der initialen farnkrautartigen Lymphgefäßen herbeiführen und bei dem zusätzliche Kompressions- und Irritationseinflüsse auf die peripheren sensiblen Nerven ausgeübt werden, so dass diese Patienten eine extrem schmerzhafte Berührungsempfindlichkeit aufweisen. Im Verlaufe von mehreren Jahren bis Jahrzehnten bilden sich unregelmäßige, unter der während des Alterungsprozesses dünner werdenden Dermis disseminiert lokalisierte Fettknötchen, welche teils schmerzhaften und stark dysästhetischen Charakter haben.

Der **Madelung'sche Fetthals** (Lanois-Bensaude-Syndrom) ist eine fettgewebsproliferierende Fettgewebsentzündung, bei der es neben einer dystrophen Fettgewebstumorausbildung auch zu einer narbenartigen Bindegewebsverdichtung im Subkutanraum kommt. Hierbei können operative Vorgehensweisen oft nur Teilerfolge erzielen, da essentielle anatomische Strukturen in diesem Prozess mit eingeschlossen sind und die Erkrankung sich im wesentlich im Kopf-, Hals- und Schulterbereich manifestiert.

Das **Lipödem** ist eine schmerzhafte Schwellung des Fettgewebes, die besonders an den Unterschenkeln von Frauen auftritt und mit zunehmenden Alter einen progredienten Verlauf bzw. Charakter aufweist.

**Piezogene Knötchen** sind druckbedingte Handkantenknötchen und Fersenknötchen, die als multiple Fettgewebshernien, vorwiegend im medialen Bereich der Ferse bei Adipösen auftreten. Es sind in der Regel Defekte in der Septierung des subkutanen Fettgewebes, die von Patienten kosmetisch oder funktionell störend empfunden werden.

**Xanthelasma** ist eine hellgelbe, leicht erhabene, plattenförmige Ablagerung von Cholesterin im Bereich der Augenlider. Sie sind weich und leicht verschiebbar und kommen meistens an beiden Augen symmetrisch vor. Es entsteht aufgrund von lokalen Fettstoffwechselstörungen. Besonders häufig sind Frauen nach den Wechseljahren betroffen. Auch bei Diabetes mellitus und bei erhöhten Blutfettwerten besteht ein erhöhtes Risiko, es zu entwickeln. Xanthelasmen können wegen ihres Aussehens psychisch belastend sein.

Verschiedene Formen der **Lipodystrophie,** wie Lipodystrophy Syndrom, welches bei HIV-Patienten nach Behandlung mit Proteasehemmern auftreten kann, Dystrophia adiposogenitalis, welches eine endokrine Störung bei heranwachsenden Mädchen ist, Sphingolipidosen, welche meist hereditären Charakter haben wie Angiokeratoma corpis (Fabry Syndrom) oder Gangliosidosen mit kutaner Manifestation.

Unter dem Begriff "Regression" wird die Lipolyse des Fettgewebes und Rückbildung des proliferierten Fettbereichs verstanden.

Die oben genannten Fettgewebserkrankungen zeigen im Gegensatz zu der alimentär bedingten Adipositas-korrelierten Lipohypertrophie pathologisch eindeutig abzugrenzende Gewebszustände oder Identitäten, die sich durch histologische Vernarbungs- und Entzündungsparameter darstellen lassen, aber auch durch Bindegewebsabkapselungen und durch Veränderungen in der histologischen Fettgewebsmorphologie selbst.

Die Erfindung betrifft auch die Verwendung von wässrigen Phospholipidsystemen, enthaltend
a) mindestens ein Phospholipid,
b) mindestens eine Gallensäure und
c) Wasser
zur Herstellung eines Arzneimittels zur Behandlung von Cellulite.

Die **Cellulite** ist eine Sonderform der hypertrophen Proliferation von Fettgewebe, welches sich zwischen der dermalen Fettfaszie (Kampa'sche Fettfaszie) und der Unterseite der Dermis befindet. Durch hormonelle Einflüsse kommt es zur verstärkten Wasserbindungskapazität dieser Fettzellen, die selbst wiederum durch Druckphänomene Lymphbahnstauungen im Bereich der initialen farnkrautartigen Lymphgefäßen herbeiführen. Im Verlaufe von mehreren Jahren bis Jahrzehnten bilden sich unregelmäßige, unter der während des Alterungsprozesses dünner werdenden Dermis disseminiert lokalisierte Fettknötchen, die teils schmerzhaften und stark dysästhetischen Charakter haben.

Die Erfindung betrifft auch die Verwendung von mindestens einem Phospholipid oder mindestens einer Gallensäure zur Herstellung eines Arzneimittels zur Behandlung von Fettgewebserkrankungen oder Cellulite.
Werden nur Phospholipid oder Gallensäure alleine eingesetzt so gelten die gleichen Bedingungen und Definitionen wie für die oben genannten Mischungen aus Phospholipid und Gallensäure.

Die Erfindung betrifft auch den Einsatz von Phospholipid, worin das Phospholipid als physiologisch verträgliches Salz vorliegt, beispielsweise als Natrium-, Kalium- und/oder Ammoniumsalz.

Das Phospholipid kann aus Ölsaaten, Raps, Sojabohne oder Sonnenblumen isoliert und nach entsprechender Reinigung im Liposomensystem eingesetzt werden. Auch Lecithin beispielsweise aus dem Hühnerei ist geeignet. Bevorzugt sind Phospholipide aus Sojabohnen.
Die Erfindung betrifft auch den Einsatz von Phospholipid, worin das Phospholipid das Phosphatidylcholin der Sojabohne ist und aus ihr isoliert wird. Insbesondere dann, wenn das Phospholipid zu mindestens 90 Gewichtsprozent (Gew. %) aus Phosphatidylcholin der Sojabohne, insbesondere zu 95 Gew. %, besteht.

Die Erfindung betrifft auch den Einsatz von einer Gallensäure, worin die Gallensäure als physiologisch verträgliches Salz vorliegt. Hierbei handelt es sich beispielsweise um ein Natrium-, Kalium und/oder Ammonium-Salz der Desoxycholsäure, Cholsäure, Lithocholsäure, Chenodesocycholsäure, Hyodesoxycholsäure, Trihydroxykoprostansäure, Ursodesoxycholsäure, Taurocholsäure oder Glykocholsäure.

Das Massenverhältnis von Phospholipid zur Gallensäure beträgt in Gew. % von 30 : 1 bis zu 1 : 0,03, bevorzugt von 1 : 0,7 bis zu 1: 0,1, insbesondere 1 : 0,6 bis zu 1 : 0,3.
Die Phospholipid Konzentration in dem Liposomensystem beträgt von 0,5 Gew. % bis 30 Gew. %, bevorzugt von 5 Gew. % bis 25 Gew. %, insbesondere von 10 Gew. % bis 20 Gew. %.
Die Liposomen haben einen Durchmesser von 30 nm bis 180 nm, bevorzugt von 30 nm bis 130 nm, insbesondere von 50 nm bis 90 nm. Diese Liposomen lassen sich problemlos steril filtrieren, wobei dann Filter mit einem Porendurchmesser von 0,2 µm eingesetzt werden.
Der pH-Wert des Liposomensystems liegt um den Neutralpunkt, vorzugsweise von 5,0 bis 8,0, insbesondere von 6,2 bis 7,4.

Die Herstellung des Liposomensystems erfolgt beispielsweise dadurch, dass man mindestens ein Phospholipid und mindestens eine Gallensäure im oben genannten Verhältnis zueinander in einem organischen Lösungsmittel löst oder dispergiert.

Anschließend wird diese Lösung oder Dispersion eingeengt und danach wird Wasser zugegeben, wobei sich das Liposomensystem ausbildet. Die Herstellung des Liposomensystems kann nach Zugabe des Wassers durch Extrusion, Hochdruckspalthomogenisation und/oder Ultraschallbehandlung gefördert werden. Die Behandlung erfolgt unterhalb von 40 °C, bevorzugt von 20 °C bis 30 °C. Geeignete organische Lösungsmittel sind Ethanol, Propanol, Isopropylalkohol oder Benzylalkohol jeweils allein oder in Mischung. Die Restvolumina an Alkoholen nach Einengung sollten von 0 Volumenprozent (Vol. %) bis zu 20 Vol. %, bevorzugt von 0 Vol % bis 10 Vol. %, betragen.
Verfahren zur Herstellung des Liposomensystems werden auch in den Europäischen Patentanmeldungen EP 0 470 437 oder EP 0 615 746 beschrieben.

Die Applikation des erfindungsgemäß eingesetzten Liposomensystems erfolgt durch subkutane, intra-artikuläre, intraperitoneale, intramuskuläre oder intravenöse Injektion. Bevorzugt ist die subkutane Injektion.
Weiterhin ist eine perkutane Applikation in verschiedenen Trägermedien und unter Einsatz verschiedener Hilfsmittel, beispielsweise lontophorese, möglich.
Die gleichmäßige Einbringung des erfindungsgemäß eingesetzten Liposomensystems sollte über ein Tumeszenzverfahren erfolgen, welches sich des hydrostatischen Drucks bedient, um eine gleichmäßige Verteilung zu gewährleisten.

Geeignete Zubereitungsformen sind beispielsweise Suspensionen, Emulsionen oder injizierbare Lösungen sowie Präparate mit protrahierter Wirkstoff-Freigabe, bei deren Herstellung übliche Hilfsmittel wie Verwendung finden.

Vorzugsweise werden die pharmazeutischen Präparate in Dosierungseinheiten hergestellt und verabreicht, wobei jede Einheit als aktiven Bestandteil eine bestimmte Dosis des Liposomensystems enthält. Bei Injektionslösungen in Ampullenform, kann diese Dosis von etwa 10 mg bis zu etwa 2000 mg, bevorzugt von etwa 50 mg bis zu etwa 2000 mg, vorzugsweise von etwa 250 mg bis 500 mg bezogen auf das Phospholipid, betragen.

Für die Behandlung eines erwachsenen Patienten sind je nach Größe des behandelten Fettgewebes bei der Applikation von Injektionslösungen Tagesdosen von 5 mg bis 500 mg, bevorzug 250 mg bis 500 mg pro Injektion bezogen auf das Phospholipid notwendig. Die Injektionslösungen können vor Applikation auch noch verdünnt werden, vorzugsweise mit Kochsalzlösung. Unter Umständen können jedoch auch höhere oder niedrigere Tagesdosen angebracht sein. Die Dosis ist auch abhängig von der Größe der Lipome, bei kleinen Lipomen reichen Mengen von 1 mg bis 50 mg, bevorzugt 2 mg bis 20 mg, pro Injektion bezogen auf das Phospholipid völlig aus. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Nachfolgend ist die Erfindung an Hand von Beispielen näher erläutert.

### Beispiele:

### Beispiel 1 Herstellung des Liposomensystems

250 g hochreines Sojabohnenphosphatidylcholin, welches mehr als 90 Gew. % Phosphatidylcholin enthält, und 126,5 g Desoxycholsäure wurden in 1 Liter Ethanol gelöst. Anschließend wurde die erhaltene Lösung unter verminderten Druck bis zur Trockenheit eingeengt. Der erhaltene Rückstand wurde in 5 Liter Wasser dispergiert und danach mittels Hochdruckspalthomogenisation auf einen mittleren Liposomendurchmesser von 30 nm bis
100 nm gebracht. Danach wurde das entstandene Liposomensystem unter sterilen Bedingungen über ein 0,2 µm Filter filtriert und unter sterilen Bedingungen in Ampullen zu jeweils 5 mL Liposomensystem abgefüllt.

### Beispiel 2 Regression von Lipomen

### a) Die Patientin kam zur Vorstellung mit der Frage einer Liposuktion des Abdomens und im Rahmen dieser Behandlung wurde folgende Anamnese erhoben:

In der Jugend und im jungen Erwachsenenalter war sie Akrobatin und hat geräteturnartige körperliche Bewegungen ausgeübt. Dabei kam es zu einem stumpfen Trauma mit einem starken Bluterguss unterhalb des linken Schulterblattes. Im weiteren Verlauf kam es zu einer, insbesondere bei besonderen Bewegungen, stärkeren Abhebung des Schulterblattes durch einen Gewebstumor, der über viele Jahre konstant geblieben ist.

Im Rahmen der Behandlung wurde dann die Frage der therapeutischen Möglichkeit der Entfernung besprochen und der Patientin wurde eine Lipomexstirpation mit Hilfe der Tumeszenzlokalanästhesie empfohlen. Die im weiteren Verlauf durchgeführte Teilexstirpation verlief problemlos, jedoch unvollständig. Die akut eingetretene Verbesserung schwächte sich ab und es kam zu einer teilweisen Rückbildung des Prozesses unterhalb des Schulterblattes, welche dann computertomografisch (CT) untersucht wurde.

Im radiologischen Befund ergeht folgende Beurteilung: Keine knöchernen Veränderungen an der Scapula, im CT besteht der Verdacht auf ein deutliches Restgewebe am medialen Scapula-Unterrand in der Loge des Musculus trapezius. Dieser Muskel ist aufgetrieben und lipomatös durchsetzt, differentialdiagnostisch Tumorrest. Ergänzende Magnetresonanztomografie gemäß obiger Ausführungen empfohlen, insbesondere wenn der Vorsatz zu einer erneuten Operation besteht.

Anschließend wurde bei der erneuten Vorstellung der Patientin etwa 4 Monate nach der Operation eine Infiltration mit 5 mL Lipostabil® N i.v.(Rote Liste, März 2003) mit einer 10 cm langen Kanüle vorgenommen und in diesem Befund verteilt. Die Patientin berichtete über einen Tag lang leicht brennende Empfindungen, die jedoch dann verschwanden. Es kam relativ zügig zu einer Rückbildung des Lipoms bis zur völligen Beschwerdefreiheit.

Danach blieb die Patientin beschwerdefrei.

### b) Der Patient hatte ein etwa walnussgroßes Lipom am Oberarm rechts Der Patient hatte keine Fettstoffwechselstörungen und die Serumlipide waren im Normbereich.

Bei dem Patienten wurde eine Menge von 0,2 ml Lipostabil®, verdünnt mit 0,2 ml NaCl ad inj. Injiziert. Nach den ersten 10 Tagen kam es zu einer deutlichen Regression des Lipoms.

### Beispiel 3 Regression von ausgeprägter Cellulite

Die zwei Patientinnen hatten keine Fettstoffwechselstörungen und die Serumlipide waren im Normbereich.
Die beiden Patientinnen erhielten in einer Sitzung 0,4 ml Lipostabil®, verdünnt mit 0,6 ml NaCl Lösung inj. (Gesamtinjektionsmenge 1,0 ml) injiziert. Es wurden pro "Celluliteberg" 0,1 ml der Lösung injiziert und mit der gesamten Menge wurde ein etwa handtellergroßes Areal an den Oberschenkelaußenseiten beiderseits (Injektionsschema ähnlich Botoxschema bei Hyperhidrose) behandelt. Bei nur geringfügigem Druckschmerz und Berührungsempfindlichkeit und mäßigem Erythem kam es bereits in den ersten zwei Wochen zu einer Regression der erhabenen Areale. Es wurde auch eine sonographische Kontrolle durchgeführt.

## Patentansprüche

1. Verwendung von wässrigen Phospholipidsystemen, enthaltend
a) mindestens ein Phospholipid,
b) mindestens eine Gallensäure und
c) Wasser
zur Herstellung eines Arzneimittels zur Behandlung von Fettgewebserkrankungen.

2. Verwendung gemäß Anspruch 1, **dadurch gekennzeichnet, dass** als Phospholipid eine der folgenden Verbindungen 3-sn-Phosphatidylcholin, Soja (Phospholipon 90), 3-sn-Phosphatidylcholin, reduziertes Soja (Phospholipon 90H), 3-(3sn)-Phospohatidyl)glycerol Soja (Phospholipon G), Dimyristoylphosphatidylglycerol, Lyso-phosphatidylcholin oder Dipalmitoylphosphatidylglycerol sowie deren physiologisch verträglichen Salze oder eine Mischung dieser Verbindungen eingesetzt werden.

3. Verwendung gemäß Anspruch 2, **dadurch gekennzeichnet, dass** als physiologisch verträglichen Salz des Phospholipids sein Natrium-, Kalium- und/oder Ammoniumsalz eingesetzt wird.

4. Verwendung gemäß Anspruch 2, **dadurch gekennzeichnet, dass** als Phospholipid das Phosphatidylcholin der Sojabohne eingesetzt wird.

5. Verwendung gemäß Anspruch 4, **dadurch gekennzeichnet, dass** das Phospholipid zu mindestens 90 Gewichtsprozent (Gew. %) aus Phosphatidylcholin der Sojabohne, insbesondere zu 95 Gew. %, besteht.

6. Verwendung gemäß einem oder mehreren der Ansprüche 1 bis 5, **dadurch gekennzeichnet, dass** als Gallensäure eine der folgenden Verbindungen Desoxycholsäure, Cholsäure, Lithocholsäure, Chenodesocycholsäure, Hyodesoxycholsäure, Trihydroxykoprostansäure, Ursodesoxycholsäure, Taurocholsäure oder Glykocholsäure sowie deren physiologisch verträglichen Salze oder eine Mischung dieser Verbindungen eingesetzt werden.

7. Verwendung gemäß Anspruch 6, **dadurch gekennzeichnet, dass** als physiologisch verträglichen Salz der Gallensäure sein Natrium-, Kalium- und/oder Ammoniumsalz eingesetzt wird.

8. Verwendung gemäß einem oder mehreren der Ansprüche 1 bis 7, **dadurch gekennzeichnet, dass** das Massenverhältnis von Phospholipid zur Gallensäure in Gewichtsprozent von 30 : 1 bis zu 1 : 0,03, bevorzugt von 1 : 0,7 bis zu 1: 0,1, insbesondere 1 : 0,6 bis zu 1 : 0,3 beträgt.

9. Verwendung gemäß einem oder mehreren der Ansprüche 1 bis 8, **dadurch gekennzeichnet, dass** die Phospholipid-Konzentration von 0,5 Gew. % bis 30 Gew. %, bevorzugt von 5 Gew. % bis 25 Gew. %, insbesondere von 10 Gew. % bis 20 Gew. % in dem Liposomensystem beträgt.

10. Verwendung gemäß einem oder mehreren der Ansprüche 1 bis 9, **dadurch gekennzeichnet, dass** die Fettgewebserkrankung ein Lipom, Morbus Dercum, Madelung'sche Fetthals, Lipödem, Xanthelasma oder Piezogene Knötchen darstellt.

11. Verwendung gemäß einem oder mehreren der Ansprüche 1 bis 10, **dadurch gekennzeichnet, dass** das wässrigen Liposomensystem zur Regression von Fettgewebsgeschwülsten eingesetzt wird.

12. Verwendung von wässrigen Phospholipidsystemen, enthaltend
a) mindestens ein Phospholipid,
b) mindestens eine Gallensäure und
c) Wasser
zur Herstellung eines Arzneimittels zur Behandlung von Cellulite.

13. Verwendung von mindestens einem Phospholipid oder mindestens einer Gallensäure zur Herstellung eines Arzneimittels zur Behandlung von Fettgewebserkrankungen oder Cellulite.

## Claims

1. Use of aqueous phospholipid systems comprising
a) at least one phospholipid,
b) at least one bile acid and
c) water
for producing a medicament for the treatment of adipose tissue disorders.

2. Use according to Claim 1, **characterized in that** one of the following compounds 3-sn-phosphatidylcholine, soya (Phospholipon 90), 3-sn-phosphatidylcholine, reduced soya (Phospholipon 90H), 3-(3sn)-phospohatidyl)glycerol soya (Phospholipon G), dimyristoylphosphatidylglycerol, lyso-phosphatidylcholine or dipalmitoylphosphatidylglycerol and the physiologically tolerated salts thereof or a mixture of these compounds are employed as phospholipid.

3. Use according to Claim 2, **characterized in that** the sodium, potassium and/or ammonium salt of the phospholipid is employed as physiologically tolerated salt thereof.

4. Use according to Claim 2, **characterized in that** the soybean phosphatidylcholine is employed as phospholipid.

5. Use according to Claim 4, **characterized in that** the phospholipid consists of at least 90 percent by weight (% by weight) soybean phosphatidylcholine, in particular 95% by weight.

6. Use according to one or more of Claims 1 to 5, **characterized in that** one of the following compounds deoxycholic acid, cholic acid, lithocholic acid, chenodeocycholic acid, hyodeoxycholic acid, trihydroxycoprostanic acid, ursodeoxycholic acid, taurocholic acid or glycocholic acid, and the physiologically tolerated salts thereof or a mixture of these compounds are employed as bile acid.

7. Use according to Claim 6, **characterized in that** the sodium, potassium and/or ammonium salt of the bile acid is employed as physiologically tolerated salt thereof.

8. Use according to one or more of Claims 1 to 7, **characterized in that** the mass ratio of phospholipid to bile acid in percent by weight is from 30:1 to 1:0.03, preferably from 1:0.7 to 1:0.1, in particular 1:0.6 to 1:0.3.

9. Use according to one or more of Claims 1 to 8, **characterized in that** the phospholipid concentration is from 0.5% by weight to 30% by weight, preferably from 5% by weight to 25% by weight, in particular from 10% by weight to 20% by weight, in the liposome system.

10. Use according to one or more of Claims 1 to 9, **characterized in that** the adipose tissue disorder is a lipoma, Dercum's disease, Madelung's neck, lipedema, xanthelasma or piezogenic nodules.

11. Use according to one or more of Claims 1 to 10, **characterized in that** the aqueous liposome system is employed for regression of adipose tissue tumors.

12. Use of aqueous phospholipid systems comprising
a) at least one phospholipid,
b) at least one bile acid and
c) water
for producing a medicament for the treatment of cellulite.

13. Use of at least one phospholipid or at least one bile acid for producing a medicament for the treatment of adipose tissue disorders or cellulite.

## Revendications

1. Utilisation de systèmes phospholipidiques aqueux, contenant :
a) au moins un phospholipide,
b) au moins un acide biliaire et
c) de l'eau,
pour la fabrication d'un médicament destiné au traitement de maladies du tissu adipeux.

2. Utilisation selon la revendication 1, **caractérisée en ce que** l'on utilise comme phospholipide l'une des combinaisons suivantes : 3-sn-phosphatidylcholine de soja (Phospholipon 90), 3-sn-phosphatidylcholine de soja réduit (Phospholipon 90H), 3-(3sn)-phosphatidyl-glycérol de soja (Phospholipon G), dimyristoyl-phosphatidyl-glycérol, lyso-phosphatidylcholine ou dipalmitoyl-phosphatidyl-glycérol ainsi que leurs sels physiologiquement acceptables ou un mélange de ces combinaisons.

3. Utilisation selon la revendication 2, **caractérisée en ce que** l'on utilise comme sel physiologiquement acceptable du phospholipide son sel de sodium, de potassium et/ou d'ammonium.

4. Utilisation selon la revendication 2, **caractérisée en ce que** l'on utilise comme phospholipide la phosphatidylcholine du soja.

5. Utilisation selon la revendication 4, **caractérisée en ce que** le phospholipide est constitué d'au moins 90 % (pourcentage pondéral) de phosphatidylcholine de soja, notamment de 95 % de celle-ci.

6. Utilisation selon l'une ou plusieurs des revendications 1 à 5, **caractérisée en ce que** l'on utilise comme acide biliaire l'une des combinaisons suivantes : acide désoxycholique, acide cholique, acide lithocholique, acide chénodésoxycholique, acide hyodésoxycholique, acide trihydroxycoprostanique, acide ursodésoxycholique, acide taurocholique ou acide glycocholique ainsi que leurs sels physiologiquement acceptables ou un mélange de ces combinaisons.

7. Utilisation selon la revendication 6, **caractérisée en ce que** l'on utilise comme sel physiologiquement acceptable de l'acide biliaire son sel de sodium, de potassium et/ou d'ammonium.

8. Utilisation selon une ou plusieurs des revendications 1 à 7, **caractérisée en ce que** le rapport massique du phospholipide à l'acide biliaire en pourcentage pondéral vaut de 30 : 1 à 1 : 0,03, de préférence de 1 : 0,7 à 1 : 0,1, notamment de 1 : 0,6 à 1 : 0,3.

9. Utilisation selon une ou plusieurs des revendications 1 à 8, **caractérisée en ce que** la concentration de phospholipide vaut en pourcentage pondéral de 0,5 % à 30 %, de préférence de 5 % à 25 %, notamment de 10 % à 20 %, dans le système liposomique.

10. Utilisation selon une ou plusieurs des revendications 1 à 9, **caractérisée en ce que** la maladie du tissu adipeux est un lipome, une maladie de Dercum, une lipomatose cervicale de Madelung, un lipoedeme, un xanthélasma ou des papules piézogéniques.

11. Utilisation selon une ou plusieurs des revendications 1 à 10, **caractérisée en ce que** le système liposomique aqueux est utilisé pour la diminution de tumeurs du tissu adipeux.

12. Utilisation de systèmes phospholipidiques aqueux, contenant :
a) au moins un phospholipide,
b) au moins un acide biliaire et
c) de l'eau,
pour la fabrication d'un médicament destiné au traitement de la cellulite.

13. Utilisation d'au moins un phospholipide ou d'au moins un acide biliaire pour la fabrication d'un médicament destiné au traitement de maladies du tissu adipeux ou de la cellulite.
